(19) Europäisches Patentamt
European Patent Office
Office européen des brevets

(11) Veröffentlichungsnummer: **0 372 415**
**A1**

(12) **EUROPÄISCHE PATENTANMELDUNG**

(21) Anmeldenummer: **89122200.2**

(22) Anmeldetag: **01.12.89**

(51) Int. Cl.⁵: **C07C 311/48, C07C 303/42, C01B 11/06**

(30) Priorität: **03.12.88 DE 3840814**

(43) Veröffentlichungstag der Anmeldung:
**13.06.90 Patentblatt 90/24**

(84) Benannte Vertragsstaaten:
**AT BE CH DE ES FR GB IT LI LU NL SE**

(71) Anmelder: **BEHRINGWERKE Aktiengesellschaft**
**Postfach 1140**
**D-3550 Marburg 1(DE)**

(72) Erfinder: **Stief, Thomas, Dr.**
**Avda. Kansas City 28. Bloque, 1. Apt. 225**
**E-41007 Sevilla(ES)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) **Verfahren zur Stabilisierung von Chloraminen oder Salzen von unterchloriger Säure.**

(57) Es wird ein Verfahren zur Stabilisierung von Chloraminen mittels einer oxidationsresistenten Puffersubstanz beschrieben.

EP 0 372 415 A1

## Verfahren zur Stabilisierung von Chloraminen oder Salzen von unterchloriger Säure

Die Erfindung betrifft ein Verfahren zur Stabilisierung von Chloraminen oder Salzen von unterchloriger Säure mittels oxidationsresistenter Puffersubstanzen.

Chloramine als Methionin zu Methioninsulfoxid oxidierende Agenten sind in der klinischen Chemie der Fibrinolyse von Bedeutung. Sie ermöglichen eine von anwesenden alpha-2-Antiplasmin nahezu unabhängige Messung fibrinolytischer Aktivität.

Lösungen von Chloraminen sind jedoch nur begrenzte Zeit haltbar, insbesondere wenn oxidierbare Substanzen anwesend sind. Zweckmäßig ist daher, die Chloramine in lyophilisierter Form bereitzustellen. Gebräuchliche Lyophilisationsfüllstoffe wie Mannit sind jedoch ungeeignet, da sie mit Oxidantien reagieren.

Aufgabe der Erfindung ist daher, ein Verfahren zur Stabilisierung von Chloraminen zur Verfügung zu stellen.

Oxidantien vom Typ der Chloramine wie Chloramin T, Chloramin B, HOCl oder deren Salze in gelöster oder getrockneter, vorzugsweise lyophilisierter Form werden überraschenderweise durch Zusatz von oxidationsinerten Puffern, die bei pH 7-12, vorzugsweise 8-11 puffern, wie N-(2-hydroxyethyl )piperazin-N-3-propansulfonsäure (HEPPS) oder N,N'-Bis(2-hydroxyethyl)glycin (BICIN) stabilisiert.

Gegenstand der Erfindung ist daher ein Verfahren zur Stabilisierung von Chloraminen, dadurch gekennzeichnet, daß eine oxidationsresistente Puffersubstanz zugegeben wird.

Bevorzugt ist BICIN.

Die Puffer werden in Konzentrationen von 1-500, vorzugsweise 5-50 mM verwendet.

Derart stabilisierte Chloramine behalten in getrockneter Form ihre Oxidationskraft, selbst wenn sie länger als 2 Wochen bei 37°C gelagert werden. In gelöster Form behalten die derart stabilisierten Chloramine über längere Zeit einen eingestellten alkalischen pH-Wert.

Die Erfindung wird weiterhin in den Ansprüchen definiert und wird in den folgenden Beispielen erläutert.

Beispiel 1

Stabilisierung von Chloramin T-Lösungen durch Pufferzusatz

Der Inhalt je eines Reagenzfläschchens "Oxidant" aus einem PAI(Plasminogenaktivator-Inhibitor)-Test-Kits, Behringwerke Marburg, FRG, wurde in 10 ml aqua dest., Tris-, TAPS -, HEPPS- oder BICIN-Puffer (alle 10 mM, pH 9.5) gelöst. Die Konzentrationen in diesen Lösungen sind dann: 8 mM Chloramin T, 3 mM Tranexamsäure (trans-4-Aminomethyl-Cyclohexan-Carbonsäure), 3% Ficoll. Nach verschiedenen Zeitintervallen bei 37°C wurde mit einem Standard-Human-Plasma, das durch Immunadsorption von PAI befreit wurde ("PAI-Mangelplasma"; "Standard S 1") ein PAI-Test (Thromb. Res. 50, 559, 1988) durchgeführt. Ergebnis siehe Tabelle 1.

### Tabelle 1

| Chloramin T-Lyophilisat aufgelöst in | gemessene Plasminaktivität (A/5 min) mit Standard S 1 nach Lagerung von | | | |
|---|---|---|---|---|
| | 0 Tag | 0,25 Tagen | 1 Tag | 7 Tagen |
| aqua dest. | 1.052 | 1.062 | 1.133 | 1.052 |
| Tris* | 1.067 | 1.093 | 1.112 | 0.110 |
| TAPS** | 1.067 | 1.109 | 1.111 | 0.219 |
| HEPPS | 1.062 | 1.085 | 1.130 | 1.051 |
| BICIN | 1.064 | 1.094 | 1.127 | 1.090 |

\* Tris(hydroxymethyl)-aminomethan
\*\* N-Tris(hydroxymethyl)methyl-aminopropansulfonsäure

Man erkennt, daß Tris und TAPS zu einem Verlust an gemessener Plasminaktivität in Abhängigkeit von der Lagerungszeit führt.

Beispiel 2

Beispiel 1 wurde statt mit Chloramin T mit Chloramin B wiederholt (Tabelle 2).

Tabelle 2

| Chloramin B-Lyophilisat aufgelöst in | gemessene Plasminaktivität (A/5 min) mit Standard S 1 nach Lagerung von | | | |
|---|---|---|---|---|
| | 0 Tag | 0,25 Tagen | 1 Tag | 7 Tagen |
| aqua dest. | 1.082 | 1.124 | 1.117 | 1.135 |
| Tris | 1.102 | 1.119 | 1.034 | 0.442 |
| TAPS | 1.099 | 1.119 | 1.067 | 0.779 |
| HEPPS | 1.093 | 1.116 | 1.110 | 1.170 |
| BICIN | 1.082 | 1.112 | 1.102 | 1.171 |

Man erkennt, daß auch hier BICIN oder HEPPS die Chloraminaktivität stabilisieren.

**Ansprüche**

1. Verfahren zur Stabilisierung von Chloraminen oder Salzen von unterchloriger Säure, dadurch gekennzeichnet, daß eine oxidationsresistente Puffersubstanz zugegeben wird.

2. Verfahren nach Anspruch 1, dadurch gekennzeichnet, daß die Substanz N-(2-hydroxyethyl)piperazin-N-3-propansulfonsäure (HEPPS) oder N,N'-Bis(2-hydroxyethyl)glycin (BICIN) ist.

3. Verfahren nach mindestens einem der Ansprüche 1 und 2, dadurch gekennzeichnet, daß die Substanzen in Konzentrationen von 0.3-30 Gewichtsprozent verwendet werden.

4. Verfahren nach mindestens einem der Ansprüche 1 bis 3, dadurch gekennzeichnet, daß getrocknet wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-3 767 586 (A.F. RUTKIEWIC) <br> * Ansprüch 1 * <br> --- | 1 | C 07 C 311/48 <br> C 07 C 303/42 <br> C 01 B 11/06 |
| X | FR-A-2 087 248 (DU PONT) <br> * Ansprüche 1-8 * <br> --- | 1 | |
| A | DE-C- 402 983 (CHEMISCHE FABRIK VON HEYDEN) <br> --- | | |
| A | US-A-1 784 286 (P.R. HERSHMAN) <br> --- | | |
| A | CHEMICAL ABSTRACTS, Band 99, 1983, Seite 123, Zusammenfassung Nr. 107499, Columbus, Ohio, US; & ES-A-510 173 <br> ----- | | |
| | | | **RECHERCHIERTE SACHGEBIETE (Int. Cl.5)** |
| | | | C 07 C 311/00 <br> C 01 B 11/00 |

Der vorliegende Recherchenbericht wurde für alle Patentansprüche erstellt

| Recherchenort | Abschlußdatum der Recherche | Prüfer |
|---|---|---|
| DEN HAAG | 12-02-1990 | ZAROKOSTAS K. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument